# EUROPÄISCHE PATENTANMELDUNG

(11) **EP 1 136 034 A1**
(43) Veröffentlichungstag der Anmeldung: **26.09.2001**
(21) Anmeldenummer: 01106595.0
(22) Anmeldetag: 15.03.2001
(51) Int. Cl.: A61B 5/00

(54) **Vorrichtung zum Erkennen von fluoreszierenden Substanzen an Zähnen**

(30) Priorität: 17.03.2000 DE 10013210
(71) Anmelder: KALTENBACH & VOIGT GMBH & CO., 88400 Biberach (DE)
(72) Erfinder: Hack, Alexander, 88400 Biberach-Rissegg (DE)
(74) Vertreter: Schmidt-Evers, Jürgen, Dipl.-Ing.

(57) **Zusammenfassung**

Bei einer Vorrichtung zum Erkennen von Karies, Plaque, bakteriellem Befall, Konkrementen, Zahnstein und anderen fluoreszierenden Substanzen an Zähnen, mit Mitteln (2) zum Erzeugen einer Anregungsstrahlung (A), welche auf einen zu untersuchenden Zahngewebebereich zu lenken ist, und Erfassungsmitteln (7) und Auswertemitteln zum Erfassen und Bewerten einer von dem bestrahlten Zahngewebebereich als Antwort auf die Bestrahlung erzeugten Fluoreszenzstrahlung (F), wird ein in dem optischen Weg zwischen den Mittel (2) zum Erzeugen der Anregungsstrahlung (A) und dem zu untersuchenden Zahngewebebereich angeordneter Strahlteiler, der die Anregungsstrahlung (A) in Richtung des Zahngewebebereichs reflektiert und die Fluoreszenzstrahlung (F) im wesentlichen durchläßt, durch die plane Rückseite (4) einer im wesentlichen hemisphärischen Linse (3) gebildet wird. Hierdurch wird eine deutlich kompaktere optische Diagnosevorrichtung ermöglicht.

## Beschreibung

Die vorliegende Erfindung betrifft eine Vorrichtung zum Erkennen von Karies, Plaque, bakteriellem Befall, Konkrementen, Zahnstein und anderen fluoreszierenden Substanzen an Zähnen nach dem Oberbegriff des Anspruches 1.

Derartige Vorrichtungen, mit deren Hilfe berührungslos das Vorhandensein von Karies an Zähnen festgestellt werden kann, wurden in letzter Zeit mehrfach vorgeschlagen. Dabei wird ein zu untersuchender Zahngewebebereich mit einer - im Idealfall - monochromatischen Lichtquelle bestrahlt und an dem bestrahlten Bereich als Antwort darauf eine Fluoreszenzstrahlung erzeugt. Diese Fluoreszenzstrahlung wird anschließend ausgewertet, wobei der Umstand ausgenützt wird, daß das Fluoreszenzspektrum von kariösem Zahngewebebereich einen signifikanten Unterschied zu dem entsprechenden Spektrum eines gesunden Zahngewebebereichs aufweist. So ist beispielsweise im roten Spektralbereich (ca. 550 bis 650 nm) eines von Karies oder Plaque befallenen Zahnes die Fluoreszenzintensität deutlich höher als bei einem gesunden Zahn. Durch eine geeignete Erfassung und Auswertung kann damit ein kariöser Zahngewebebereich eindeutig von einem gesunden Zahngewebebereich mit Hilfe eines einfachen und berührungslosen Untersuchungsverfahrens unterschieden werden.

Vorrichtungen dieser Art werden beispielsweise in der DE 297 04 185 U1 oder der DE 197 09 500 C1 beschrieben. Sie weisen zunächst eine Einrichtung zum Erzeugen der Anregungsstrahlung auf, beispielsweise einen HeNe-Laser, der eine Anregungsstrahlung mit einer Wellenlänge im Bereich zwischen 600 nm und 670 nm erzeugt. Die Anregungsstrahlung wird über ein optisches System, welches aus mehreren Linsen oder Spiegeln besteht, in ein Lichtleitersystem eingekoppelt und durch dieses auf den zu untersuchenden Zahngewebebereich gerichtet. Bei der in der DE 197 09 500 C1 beschriebenen Vorrichtung besteht das Lichtleitersystem aus einem sich von der Lichtquelle bis zu der Spitze eines Handstücks erstreckenden Lichtleiter, an dessen Ende eine Lichtsonde angebracht ist, durch die das Licht ausgekoppelt und auf den gewünschten Bereich gerichtet wird. Die an dem bestrahlten Zahngewebebereich entstehende Fluoreszenzstrahlung wird über die Spitze der Lichtsonde wieder in das Lichtleitersystem eingekoppelt und zu einer Erfassungsvorrichtung übertragen. Da üblicherweise das Lichtleitersystem gleichzeitig zum Übertragen sowohl der Anregungsstrahlung als auch der Fluoreszenzstrahlung verwendet wird, befindet sich in dem optischen Weg zwischen der Anregungsstrahlungsquelle und dem zu untersuchenden Zahngewebebereich ein Strahlteiler, um beide Strahlungsarten voneinander zu trennen und die Fluoreszenzstrahlung den Erfassungsmitteln zuzuführen.

Die zuvor beschriebenen Elemente der optischen Diagnosevorrichtung, insbesondere die Mittel zum Erzeugen der Anregungsstrahlung, das optische System einschließlich des Strahlteilers zum Einkoppeln der Anregungsstrahlung in das Lichtleitersystem bzw. zum Auskoppeln der Fluoreszenzstrahlung weisen eine gewisse Größe auf, so daß es bisher üblich war, diese Elemente in einer Zentraleinheit anzuordnen und die beiden Strahlungsarten zu dem bzw. von dem zahnärztlichen Handstück mit Hilfe eines Versorgungsschlauches, in dem ein oder mehrere Lichtleiter angeordnet sind, zu übertragen.

Es ist Aufgabe der vorliegenden Erfindung, die bekannten Vorrichtungen zum Erkennen von Karies, Plaque, bakteriellem Befall, Konkrementen, Zahnstein und anderen fluoreszierenden Substanzen an Zähnen derart zu verbessern, daß sie in ihrem Aufbau einfacher und platzsparender ausgeführt werden können.

Diese Aufgabe wird allgemein gemäß der Erfindung durch eine Integration von Bauteilen gelöst.

Die Aufgabe wird gemäß einem Aspekt der Erfindung durch eine Vorrichtung, welche die Merkmale des Anspruches 1 aufweist, gelöst. Die Vorrichtung zeichnet sich dadurch aus, daß der in dem optischen Weg zwischen den Mitteln zum Erzeugen der Anregungsstrahlung und dem zu untersuchenden Zahngewebebereich angeordnete Strahlteiler durch die plane Rückseite einer im wesentlichen hemisphärischen Linse gebildet wird. Diese plane Rückseite ist derart ausgebildet, daß die Anregungsstrahlung in Richtung des zu untersuchenden Zahngewebebereichs reflektiert wird, die Fluoreszenzstrahlung hingegen im wesentlichen ungehindert durch sie hindurchtritt. Da die gekrümmte Vorderseite der hemisphärischen Linse gleichzeitig eine fokussierende Wirkung hat, kann mit ihrer Hilfe die Anregungsstrahlung in das Lichtleitersystem eingekoppelt werden und dabei möglicherweise auf zusätzliche optische Mittel hierfür verzichtet werden. Die erfindungsgemäße Ausgestaltung der optischen Diagnosevorrichtung zeichnet sich somit durch ihren einfachen Aufbau und die damit verbundene Möglichkeit aus, sie sehr unempfindlich gegen äußere Einflüsse gestalten zu können. Damit ist darüber hinaus die Möglichkeit gegeben, die wesentlichen Elemente der Vorrichtung nicht wie bisher in einer von dem Handstück separaten Zentraleinheit, sondern unmittelbar in dem Handstück selbst anzuordnen.

Die strahlteilende Wirkung der planen Seite der hemisphärischen Linse wird vorzugsweise dadurch erreicht, daß diese eine Beschichtung aufweist, welche Licht mit den Frequenzen der Anregungsstrahlung reflektiert und Licht im Frequenzbereich der erzeugen Fluoreszenzstrahlung ungehindert durchläßt. Vorzugsweise befindet sich anliegend an die plane Seite der hemisphärischen Linse ein optisches Filter, um die zur Erfassung und Beurteilung der Fluoreszenzstrahlung nicht benötigten und möglicherweise störenden Anteile besser herauszufiltern. Zum Erzeugen der Anregungsstrahlung wird vorzugsweise eine Laserdiode verwendet, da diese nur sehr geringe Abmessungen aufweist und somit ohne weiteres in ein Handstück integriert werden kann. Ferner können die Erfassungsmittel eine hinter dem optischen Filter angeordnete Photodiode zur Erfassung der Fluoreszenzstrahlung aufweisen.

Auf welche Weise die Anregungsstrahlung von der Lichtquelle auf den zu untersuchenden Zahngewebebereich gelenkt wird, hängt von dem Anwendungsgebiet des optischen Diagnoseverfahrens ab. Mehrere Möglichkeiten hierfür werden im folgenden besprochen.

Die erfindungsgemäße Vorrichtung kann beispielsweise sehr gut in der Parodontaldiagnostik eingesetzt werden. Hierfür ist an dem Kopfende des Handstückes eine optische Sonde vorgesehen, welche in die Zahntasche eines zu untersuchenden Zahnes eingeführt wird. An der Sondenspitze wird die Anregungsstrahlung ausgekoppelt und auf den zu untersuchenden Zahngewebebereich gelenkt. Wie zuvor beschrieben, wird die an dem bestrahlten Zahngewebebereich entstehende Fluoreszenzstrahlung ebenfalls mit Hilfe der optischen Sonde wieder in das Lichtleitersystem eingekoppelt und auf die als Strahlteiler wirkende hemisphärische Linse und die Mittel zum Auswerten der Fluoreszenzstrahlung gelenkt. Hierdurch ist die Möglichkeit gegeben, auch den üblicherweise schwer zugänglichen und somit schwer zu untersuchenden Bereich eines Zahnstumpfes mit Hilfe des einfachen aber effektiven optischen Diagnoseverfahrens beurteilen zu können.

Vorzugsweise besteht die Sonde aus einem lichtleitenden Material und hat dabei die Form eines Kegels, eines Lichtkeils oder einer Parodontalsonde. Um nähere Information über die Tiefe der Zahntasche erhalten zu können, kann die Sonde ferner an ihrer Außenseite Markierungen oder eine Skala aufweisen. Ferner ist die Sondenspitze vorzugsweise federnd an dem Handstück gelagert, um den Benutzer die Möglichkeit zu geben, die Sondierungskraft zu dosieren und somit Verletzungen an dem Zahnfleisch des Patienten zu vermeiden. Die Übertragung der Strahlungsarten von der hemisphärischen Linse zu der optischen Sonde bzw. in entgegengesetzter Richtung kann z.B. durch einen oberhalb der Sonde angeordneten Spiegel oder einen mit dem Sondenende verbundenen flexiblen Lichtleiter erfolgen.

Wie zuvor erläutert wurde, ist ein wesentlicher Vorteil der erfindungsgemäßen Diagnosevorrichtung dahingehend zu sehen, daß sie aufgrund der Bauteilintegration in ihren Abmessungen äußerst kompakt gestaltet werden kann. Somit ist die Möglichkeit gegeben, bekannte zahnmedizinische Handstücke zum Bearbeiten eines Zahngewebebereichs durch das optische Diagnoseverfahren zu ergänzen und somit eine kombinierte Diagnose- und Behandlungsvorrichtung zu schaffen. In diesem Zusammenhang war es bisher lediglich bekannt, Laserbehandlungsgeräte mit dem optischen Diagnoseverfahren zu ergänzen, da die zur Übertragung der Laserbehandlungsstrahlung vorhandenen Lichtleiter in einfacher Weise auch von dem Diagnosesystem genutzt werden konnten. Eine derartige Diagnose- und Behandlungsvorrichtung mittels Laserstrahlung ist beispielsweise in der DE 297 05 943 U1 beschrieben.

Gemäß der vorliegenden Erfindung wird nun allerdings eine zahnmedizinische Vorrichtung mit einem Handstück zur mechanischen Bearbeitung des Zahngewebebereichs durch das optische Diagnoseverfahren ergänzt. Bei dieser zahnmedizinischen Vorrichtung kann es sich beispielsweise um ein Zahnsteinentfernungsgerät, wie es in der DE 83 22 850 U1 beschrieben ist, oder um ein Handstück, bei dem die Bearbeitung des Zahngewebebereichs durch ein abrasives Behandlungsmittel erfolgt, wie es bei beispielsweise in der DE 197 42 701 A1 beschrieben ist, handeln. Bei diesen bekannten zahnmedizinischen Vorrichtungen sind die Handstücke in der Regel durch die in ihnen angeordnete Antriebsvorrichtungen für die Behandlungsspitzen oder Übertragungskanäle für Behandlungsmedien oder dergleichen derart ausgefüllt, daß es bisher nicht möglich war, in dieses auch noch eine optische Diagnosevorrichtung ohne eine noch zu vertretene Vergrößerung des Handstücks zu integrieren. Diese Schwierigkeiten werden durch die erfindungsgemäße Vorrichtung nunmehr behoben, so daß eine sehr effektive und vielseitige zahnmedizinische Vorrichtung gebildet werden kann.

Im Fall des Zahnsteinentfernungsgeräts oder des Handinstruments zur Bearbeitung mittels einer abrasiven Flüssigkeit kann beispielsweise der zur Übertragung der Strahlungsarten benötigte Lichtleiter innerhalb der Instrumentenspitze oder an deren Außenseite verlaufen. Es ist dann die Möglichkeit gegeben, die Einrichtung mit einer Anzeigevorrichtung auszustatten, welche dem Benutzer unmittelbar zu verstehen gibt, ob der Zahngewebebereich, auf den die Instrumentenspitze gerichtet ist, kariös bzw. mit Konkrementen oder Belägen beladen ist oder nicht, so daß der Benutzer seine Behandlung mit dem Diagnoseergebnis abstimmen kann. Ferner kann auch die Möglichkeit vorgesehen sein, daß eine Steuereinrichtung automatisch die mechanische Bearbeitung in Abhängigkeit von dem Diagnoseergebnis steuert.

Im folgenden soll die Erfindung anhand der beiliegenden Zeichnungen näher erläutert werden. Es zeigen:
- Fig. 1: die erfindungsgemäße Vorrichtung zum Erkennen von Karies, Plaque oder bakteriellem Befall an Zähnen;
- Fig. 2: die Integration der erfindungsgemäßen Vorrichtung in ein zahnmedizinisches Handstück zur Parodontaldiagnostik;
- Fig. 3 und 4: Weiterbildungen der in Fig. 2 dargestellten Diagnosevorrichtung mit einer axial federnd gelagerten Sonde;
- Fig. 5 bis 9: mehrere Ausführungsbeispiele für die optische Sonde;
- Fig. 10: die Integration der optischen Diagnosevorrichtung in ein Zahnsteinentfernungsgerät;
- Fig. 11a: die Ausgestaltung der Instrumentenspitze des in Fig. 10 dargestellten Zahnsteinentfernungsgeräts;
- Fig. 11b: eine vergrößerte Darstellung der Instrumentenspitze;
- Fig. 12a: die Instrumentenspitze eines weiteren durch die optische Diagnosevorrichtung erweiterten Behandlungsgeräts; und
- Fig. 12b: eine vergrößerte Darstellung der in Fig. 12a gezeigten Instrumentenspitze.

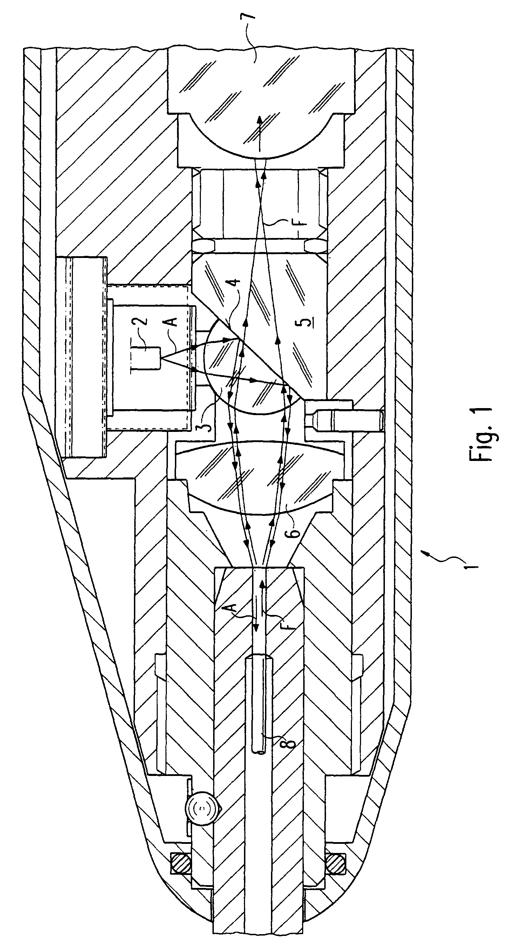

Es soll nun zunächst anhand von Fig. 1 die Anordnung der optischen Diagnosevorrichtung innerhalb eines Handstücks erläutert werden. Bei dem Handstück 1 handelt es sich um ein zunächst nicht näher definiertes zahnmedizinisches Handstück, welches einerseits ausschließlich zu Diagnosezwecken verwendet, andererseits aber auch - wie später anhand von zwei Beispielen erläutert wird - weitere Elemente zur therapeutischen Behandlung des untersuchten Zahngewebebereichs umfassen kann. Als Lichtquelle für die Anregungsstrahlung A befindet sich im oberen Bereich des Handstücks 1 eine Laserdiode 2, welche eine Anregungsstrahlung im Bereich zwischen 600 nm und 670 nm erzeugt. Vorzugsweise weist die Anregungsstrahlung eine Wellenlänge von ca. 655 nm auf, da hier der bestmögliche Kompromiß zwischen der Ausgangsleistung der Laserdiode 2 und dem spektralen Unterschied zwischen der Anregungsstrahlung A und der Fluoreszenzstrahlung F erzielbar ist.

Die von der Laserdiode 2 abgestrahlt Anregungsstrahlung A tritt in die unterhalb der Laserdiode 2 angeordnete hemisphärische Linse 3 ein und wird an der planen Rückseite 4 in Richtung zum vorderen Ende des Handstücks 1 reflektiert. Die Reflexion der Anregungsstrahlung A erfolgt dabei durch eine auf die plane Rückseite 4 der hemisphärischen Linse 3 aufgedampfte Beschichtung, welche Licht in dem zuvor erwähnten Frequenzbereich effektiv reflektiert. Beim Austritt aus der hemisphärischen Linse 3 wird die Anregungsstrahlung A durch die gekrümmte Vorderseite auf eine davor angeordnete zusätzliche Fokussierlinse 6 gelenkt, durch welche die Anregungsstrahlung A schließlich in einen zum vorderen Ende des Handstücks 1 verlaufenden Lichtleiter 8 eingekoppelt wird. Der Lichtleiter 8 führt zu einer (nicht dargestellten) optischen Sonde, einem Lichtauskoppelelement oder weiteren optischen Elementen mit deren Hilfe die Anregungsstrahlung A auf den zu untersuchenden Zahngewebebereich gelenkt wird.

Die als Antwort auf die Bestrahlung an dem Zahngewebebereich entstehende Fluoreszenzstrahlung F wird in entgegengesetzter Richtung über den Lichtleiter 8 wieder zurück zu der Fokussierlinse 6 und der hemisphärischen Linse 3 übertragen. An der planen Rückseite 4 der hemisphärischen Linse 3 wird nun allerdings die Fluoreszenzstrahlung F, deren Wellenlänge größer als diejenige der Anregungsstrahlung A ist, nicht mehr reflektiert, sondern sie tritt ungehindert hindurch in ein unmittelbar hinter der hemisphärischen Linse 3 angeordnetes optisches Filter 5. Der Sperrbereich dieses Filters 5 liegt im Wellenlängenbereich der Anregungsstrahlung A, um evtl. durch die Rückseite 4 der hemisphärischen Linse 3 hindurchtretende Anteile der Anregungsstrahlung A zu eliminieren. Dem optischen Filter 5 ist schließlich eine Photodiode 7 nachgeordnet, welche Bestandteil der Mittel zum Erfassen der Fluoreszenzstrahlung F und zum Bewerten dieser ist. Die Auswertung der Intensität der Fluoreszenzstrahlung F bzw. die Beurteilung, ob der untersuchte Zahngewebebereich Karies aufweist oder mit Konkrementen bzw. Belägen beladen ist, erfolgt entweder unmittelbar in dem Handstück 1 oder es wird ein der Intensität der Fluoreszenzstrahlung F entsprechendes Signal an eine zentrale Einheit übermittelt.

Bei dem Lichtleiter 8 kann es sich in bekannter Weise um ein Bündel aus mehreren Lichtleitfasern handeln, in deren Länge eine Faser zur Übertragung der Anregungsstrahlung A angeordnet ist, die von mehreren Erfassungslichtleitfasern für die Fluoreszenzstrahlung F umgeben ist. Die fokussierende Wirkung der gekrümmten Oberfläche der hemisphärischen Linse 3 hängt in erster Linie von der Größe und dem Radius der Linse 3 ab. Bei einer geeigneten Wahl der Abmessungen für die hemisphärische Linse 3 kann auch auf die zusätzliche Fokussierlinse 6 verzichtet werden, wodurch die optische Vorrichtung nochmals einfacher gestaltet werden kann.

Wie Fig. 1 zu entnehmen ist, besteht die Vorrichtung aus nur sehr wenigen optischen Elementen, so daß sie einerseits kostengünstig herzustellen ist, andererseits aber auch weniger anfällig gegen Erschütterungen oder Temperaturveränderungen als die bisher bekannten Diagnosevorrichtung ist.

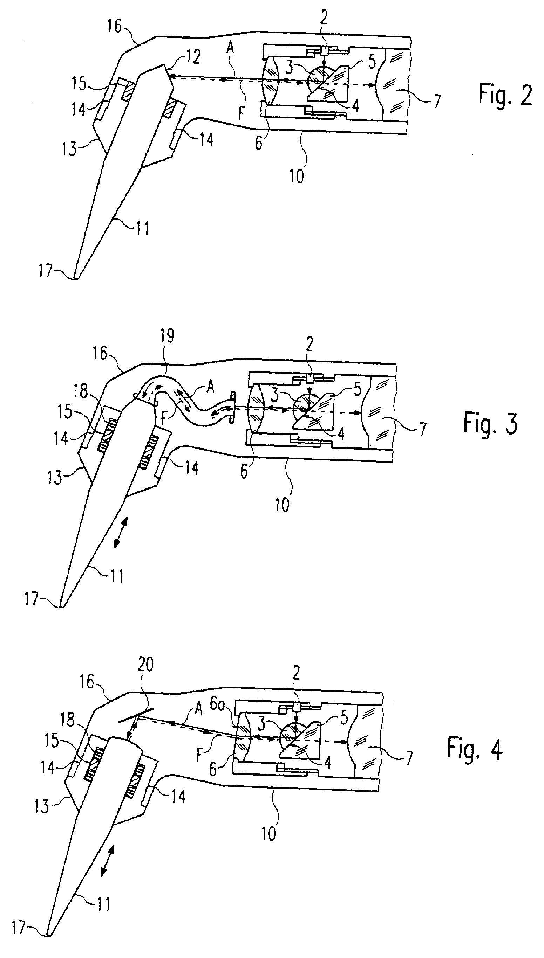

Als erstes Anwendungsbeispiel soll nun eine Vorrichtung zur Parodontaldiagnostik vorgestellt werden. Bisher waren lediglich Laserbehandlungsinstrumente bekannt, an deren Kopfende ein in einem Winkel zur Längsachse des Handstücks angeordnetes Auskoppelelement in Form einer Parodontalsonde angeordnet ist, welche in die Tasche zwischen einem Zahnstumpf und dem Zahnfleisch zur dortigen Behandlung des Zahnes, Zahnbelages oder des Zahnfleisches eingeführt werden kann. Ein derartiges Behandlungsinstrument ist beispielsweise in der DE 196 36 265 A1 beschrieben. Fig. 2 zeigt eine erfindungsgemäße Abwandlung des in der oben genannten Offenlegungsschrift beschriebenen Behandlungsinstruments zu einer Vorrichtung zu einer Parodontaldiagnostik. Hierzu wurde die anhand von Fig. 1 erläuterte optische Diagnosevorrichtung - gleiche Elemente weisen hierbei die gleichen Bezugszeichen auf - in das Handstück 10 integriert.

Das Handstück 10 weist an seinem vorderen Ende ein Kopfteil 16 auf, in dem eine in einem Winkel zur Längsachse des Handstücks 10 gelagerte optische Sonde 11 angeordnet ist. Die Lagerung der optischen Sonde 11 erfolgt über einen Schraubeinsatz 13, der über ein Gewinde 14 mit dem Kopfteil 16 verbindbar ist, wobei ein an der Außenseite der Sonde 11 angeordneter Halteansatz 15 in einer entsprechenden Ausnehmung des Schraubeinsatzes eingeschlossen wird. Diese Konstruktion bietet die Möglichkeit, die Sonde 11 schnell und auf einfache Weise durch eine neue Sonde zu ersetzen, welche beispielsweise aufgrund einer anderen Form für den neuen Untersuchungszweck besser geeignet ist.

Das Einkoppeln der Anregungsstrahlung A erfolgt mit Hilfe der zusätzlichen Fokussierlinse 6, welche die Strahlung auf eine kegelförmige Stirnfläche 12 der optischen Sonde 11 fokussiert. Die Form und das Material der Sonde 11 ist derart gewählt, daß die über die Stirnfläche 12 angekoppelte Strahlung innerhalb der Sonde 11 totalreflektiert wird und lediglich an deren unterer Spitze 17 austritt. Wie später noch erläutert wird, können die Spitzen jeweils dem zu untersuchenden Gebiet angepaßt werden.

Die unmittelbar vor der Sondenspitze 17 entstehende Fluoreszenzstrahlung F wird in zur Anregungsstrahlung A entgegengesetzter Weise durch Totalreflexion innerhalb der Sonde 11 bis zu deren oberen Stirnfläche 12 weitergeleitet und von dort auf die Fokussierlinse 6 geworfen, wonach sie in bekannter Weise über die hemisphärische Linse 3 und das optische Filter 5 auf die Photodiode gelenkt wird. Die Sonde 11 kann aus einem lichtdurchlässigen Material wie z.B. Glas bzw. Quarz oder Kunststoff bestehen. Aufgrund ihrer geringen Abmessungen und Formen ist sie insbesondere dafür geeignet, in Zahntaschen zwischen dem Zahnfleisch und der Zahnbasis eingeführt zu werden.

Fig. 3 zeigt eine Weiterbildung des in Fig. 2 dargestellten Handstücks 10, dies sich im wesentlichen dadurch unterscheidet, daß die Sonde 11 nun nicht mehr starr, sondern in Axialrichtung federnd in dem Kopfteil 16 gelagert ist. Dies wird dadurch erreicht, daß der Halteansatz 15 eine geringere Abmessung als die entsprechende Ausnehmung des Schraubeinsatzes 13 aufweist und dadurch in axialer Richtung verschiebbar ist. Um in Abwesenheit einer Kraft eine Mittelstellung der Sonde 11 zu erreichen, sind oberhalb und unterhalb des Halteansatzes 15 Federelemente 18 vorgesehen. Die federnde Lagerung der Sonde 11 gibt dem Anwender die Möglichkeit, die Sondierungskraft, also die Kraft mit der die Sonde 11 in die zu untersuchende Zahntasche eingeführt wird, feiner zu dosieren und somit unnötige Verletzungen an dem Zahnfleisch des Patienten zu vermeiden. Um in jeder Stellung der Sonde 11 ein effektives Ein- und Auskoppeln der beiden Strahlungen zu ermöglichen, wird diesmal zwischen der Fokussierlinse 6 und der Oberseite der Sonde 11 ein flexibler Lichtleiter 19 verwendet. Alternativ zu der dargestellten Lagerung könnte auch ein einziges Federelement verwendet werden, daß oberhalb des Halteansatzes 15 angeordnet ist, da die Sonde 11 in der Regel lediglich auf Druck belastet wird und eine Mittelstellung nicht unbedingt notwendig ist.

In dem in Fig. 4 dargestellten Ausführungsbeispiel ist die Sonde 11 auf gleiche Weise wie in Fig. 2 axial federnd gelagert. Allerdings wird nunmehr nicht ein flexibler Lichtleiter 19 verwendet, das Einkoppeln der Anregungsstrahlung A erfolgt hier über einen in dem Kopfteil 16 in axialer Richtung oberhalb der Sonde 11 angeordneten Spiegel 20. Zu diesem Zweck ist die dem Kopfteil 16 des Handstücks 10 zugewandte Vorderseite 6a der Fokussierlinse 6 plan und leicht geneigt, um die Anregungsstrahlung auf den Spiegel 21 umzulenken.

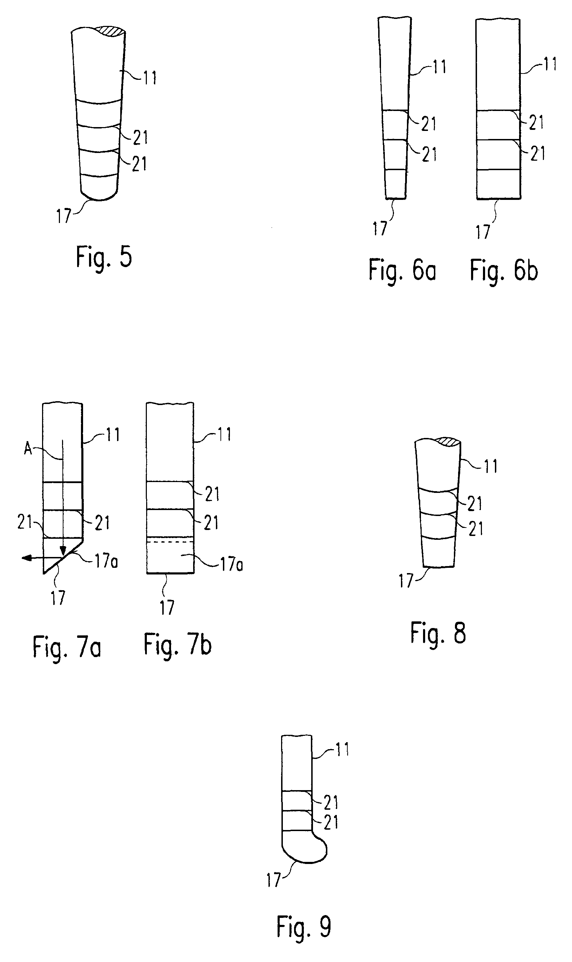

Die Figuren 5 bis 9 zeigen verschiedene Ausgestaltungsmöglichkeiten für die Sonde 11. Es ist hierbei jeweils das untere Ende der Sonde 11 dargestellt, die genauere Ausgestaltung des oberen Endes richtet sich nach der Halterung der Sonde 11 innerhalb des Kopfstücks 16 bzw. den Mitteln zum Übertragen der Anregungsstrahlung A und der Fluoreszenzstrahlung F. Die in Fig. 5 dargestellte Sonde 11 ist in ihrem unteren Bereich leicht kegelförmig und weist eine abgerundete Sondenspitze 17 auf. Von dieser Spitze 17 aus nach oben befinden sich in regelmäßigen Abständen Markierungen 21, welche nach dem Einführen der Sonde 11 in die Zahntasche darüber Auskunft geben, wie tief die Tasche ist bzw. wie weit die Sonde 11 bereits eingeführt wurde. Bei den Markierungen 21 kann es sich beispielsweise um Kerben oder auch um aufgedruckte Ringe handeln, welche die Sonde 11 vollständig umgeben und somit von jeder Richtung aus sichtbar sind.

Die in Fig. 6a in seitlicher Ansicht dargestellte Sonde 11 hat die Form eines sich zur Spitze 17 hin verjüngenden Keils. Entsprechend der Frontalansicht in Fig. 6b bleibt die Breite des Keils über seine Höhe hin unverändert. Wie auch bei der in Fig. 5 dargestellten Sonde 11 wird die Anregungsstrahlung an der Sondenspitze 17 aus bzw. die Fluoreszenzstrahlung wird über die Sondenspitze 17 wieder in die Sonde 11 eingekoppelt. Die Keilform erleichtert dabei das Einführen der Sonde in die Zahntasche. Auch hier sind die zuvor erwähnten Höhenmarkierungen 21 vorgesehen.

Die in den Fig. 7a und 7b dargestellte Sonde 11 hat die Form eines Lichtkeils mit einer an der Spitze 17 befindlichen Schrägfläche 17a, über welche die Anregungsstrahlung nicht in axialer Richtung, sondern senkrecht dazu ausgekoppelt wird. Dies ermöglicht eine Diagnose der seitlich neben der Sonde angeordneten Zahnbereichflächen.

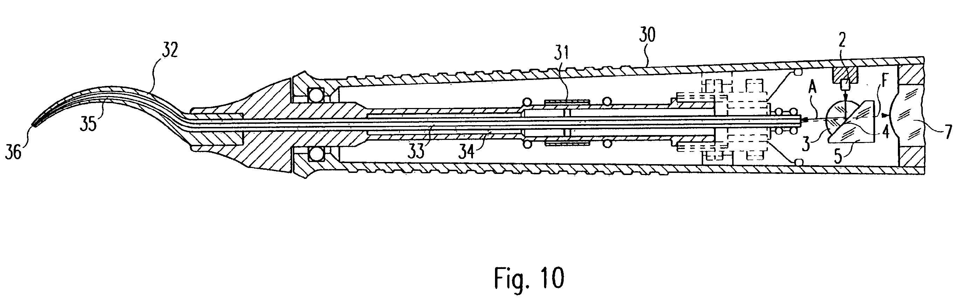

Gemäß den Figuren 8 und 9 bestehen weitere Ausgestaltungsmöglichkeiten für die Sonde 11 in einem Kegelstumpf (Fig. 8) mit einer möglicherweise verrundeten Kante an der Stirnfläche der Sondenspitze 17 oder in einer Sonde 11 mit einer abgekröpften Sondenspitze 17 zum Zugang von Furkationen (Fig. 9).

Abschließend soll nun die Ergänzung bekannter zahnärztlicher Behandlungsgeräte durch die erfindungsgemäße optische Diagnosevorrichtung erläutert werden, zunächst anhand eines Zahnsteinentfernungsgerätes, wie es in der DE 83 22 850 U1 beschrieben ist. Es sollen hier nur die wesentlichsten Elemente des Zahnsteinentfernungsgerätes beschrieben werden, da deren Funktion bereits hinlänglich aus der zuvor genannten Schrift bekannt ist. Das in Fig. 10 dargestellte Handstück 30 weist einen pneumatisch betreibbaren Schwingungserzeuger 31 auf, der dazu ausgebildet ist, die am Ende der Griffhülse 30 gehalterte Instrumentenspitze 32 in Schwingungen zu versetzen. Die Energiezuführleitung für den Schwingungserzeuger 31 ist durch eine Druckluftbildung 34 gebildet, die sich axial durch das Handstück 30 erstreckt.

Erfindungsgemäß kann das bekannte Handstück 30 dadurch ergänzt werden, indem die Elemente der optischen Diagnosevorrichtung, nämlich die Laserdiode 2, die hemisphärische Linse 3, das Fluoreszenzfilter 5 und die Photodiode 7 in einem hinteren Bereich des Handstücks angeordnet werden. Zur Übertragung der Anregungsstrahlung und der Fluoreszenzstrahlung ist ein Lichtleiter 33 vorgesehen, der sich axial innerhalb der Druckluftleitung 34 durch das Handstück 30 bis zu der Instrumentenspitze 32 erstreckt und dort in einem Kanal 35 bis zu dem auf den zu behandelnden Zahnbereich gerichteten Ende 36 verläuft. In diesem Ausführungsbeispiel wurde auf die zusätzliche Fokussierlinse verzichtet, da die Krümmung der hemisphärischen Linse 3 ausreichend ist, um die Anregungsstrahlung A in den Lichtleiter 33 einzukoppeln. Wie der Darstellung zu entnehmen ist, können die für die optische Diagnosevorrichtung wesentlichen Elemente platzsparend in dem Handstück 30 untergebracht werden.

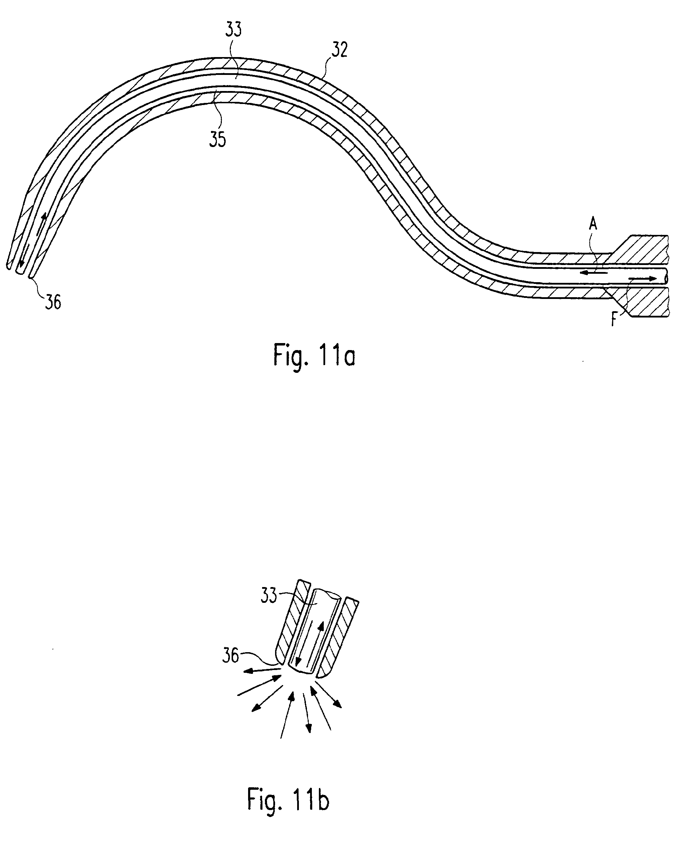

Die Ausgestaltung der Instrumentenspitze 32 ist in Vergrößerung in den Fig. 11a und 11b dargestellt. Der Lichtleiter 33 wird vorzugsweise durch sehr flexible Lichtleitfasern gebildet, um nicht durch die hochfrequenten Schwingungen der Spitze 32 beschädigt zu werden. Er erstreckt sich durch einen länglichen Hohlraum 35 der Instrumentenspitze bis zu deren Ende 36, steht allerdings nicht über dieses hervor, um nicht während des Bearbeitens des Zahngewebebereichs beschädigt zu werden. Es ist hierbei die Möglichkeit gegeben, den in der Instrumentenspitze 32 verlaufenden Kanal 35 zusätzlich auch zur Übertragung einer Kühlflüssigkeit oder dergleichen zu verwenden. Wesentlich ist, daß die Anregungsstrahlung A genau auf den Zahngewebebereich gelenkt wird, der durch die Instrumentenspitze 32 bearbeitet wird.

Die gesamte zahnärztliche Vorrichtung weist ferner eine Anzeigeeinheit auf, welche Auskunft darüber gibt, ob der Zahngewebebereich auf den die Instrumentenspitze 32 gerichtet ist, kariös bzw. mit Konkrementen oder Belägen beladen ist oder nicht, so daß ein Benutzer entsprechend dem Diagnoseergebnis mit einer therapeutischen Behandlung fortfahren kann. Eine andere Möglichkeit besteht darin, über eine Steuereinheit die Betätigung des Schwingungserzeugers 31 für die Instrumentenspitze 32 derart anzusteuern, daß das Bearbeiten durch die Instrumentenspitze 32 automatisch entsprechend dem Diagnoseergebnis durch die optische Diagnosevorrichtung erfolgt.

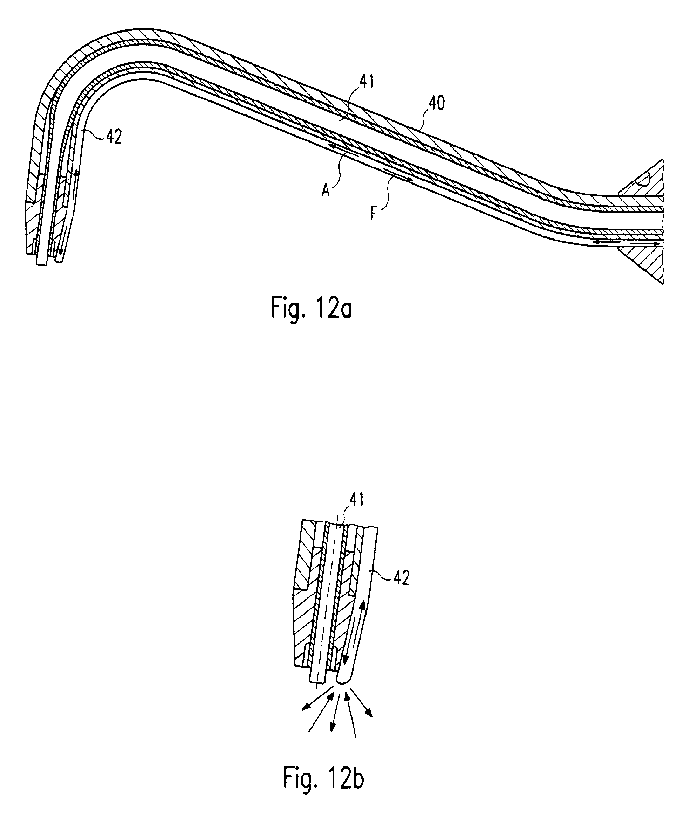

Abschließend soll die Ergänzung eines weiteren bekannten zahnmedizinischen Behandlungsgeräts durch die optische Diagnosevorrichtung vorgestellt werden. Bei dem im folgenden besprochenen Gerät handelt es sich um ein dentales Handinstrument, bei dem ein abrasives Behandlungsmittel auf den zu bearbeitenden Zahngewebebereich gelenkt wird, wie dies beispielsweise ausführlich in der DE 197 42 701 A1 beschrieben ist. Die Fig. 12a und 12b zeigen dabei die erfindungsgemäß Erweiterung der Behandlungsspitze. Das Anordnen der weiteren Elemente der Diagnosevorrichtung erfolgt analog zu dem zuvor beschriebenen Beispiel des Zahnsteinentfernungsgeräts.

Die Instrumentenspitze 40 stellt in diesem Fall eine Kanüle dar, über deren inneren Kanal 41 ein abrasives Behandlungsmittel auf einen Zahngewebebereich gerichtet wird. In diesem Fall wäre es nachteilig, den zur Übertragung der Anregungsstrahlung A und der Fluoreszenzstrahlung F verwendeten Lichtleiter 42 innerhalb dieses Kanals 41 anzuordnen, da die abrasive Flüssigkeit auch den Lichtleiter 42 beschädigen würde. Im vorliegenden Fall ist daher der Lichtleiter 42 an der Außenseite der Behandlungsspitze angeordnet, wobei wiederum die am Ende des Lichtleiters 42 ausgekoppelte Anregungsstrahlung genau auf den Bereich gerichtet ist, auf den über den Kanal 41 die abrasive Flüssigkeit aufgebracht wird. Auch in diesem Beispiel besteht die Möglichkeit, die therapeutische Funktion des Instruments abhängig von dem Diagnoseergebnis einzustellen, entweder durch eine automatische Steuervorrichtung oder durch eine optische oder akustische Anzeige des Diagnoseergebnisses.

Diese beiden Beispiele machen deutlich, daß es ohne weiteres möglich ist, bekannte Behandlungsgeräte durch das effektive optische Diagnoseverfahren zu erweitern und somit ein äußerst vielseitiges zahnmedizinisches Gerät zu bilden. Dies wird insbesondere dadurch ermöglicht, daß die Abmessungen nunmehr so gering gehalten werden können, daß die wesentlichen Elemente der optischen Diagnosevorrichtung in ein Handstück integriert werden können. Natürlich besteht auch die Möglichkeit die Mittel zum Erzeugen der Anregungsstrahlung und zum Auswerten der Fluoreszenzstrahlung in einem separaten Basisgerät unterzubringen und dann die beiden Strahlungsarten über einen Lichtleiter in einem Versorgungsschlauch für das Handstück zu übertragen. Da ferner die Möglichkeit besteht, die therapeutische Tätigkeit abhängig von dem Zustand des Zahnbereichs einzustellen, kann somit eine wesentlich bessere und effektivere Behandlung von Zahnoberflächen erzielt werden.

Schließlich ist noch anzumerken, daß grundsätzlich auch die Anordnung der Laserdiode und der Photodiode (ggf. mit dem dazugehörenden Filter) vertauscht werden kann, wobei in diesem Fall dann die reflektierende Beschichtung an der Rückseite der hemisphärischen Linse derart auszuführen ist, daß sie die Fluoreszenzstrahlung reflektiert. Die in der Zeichnung dargestellte Anordnung ist allerdings aus Gründen einer besseren Herstellbarkeit der hierfür benötigten Beschichtung vorzuziehen.

## Patentansprüche

1. Vorrichtung zum Erkennen von Karies, Plaque, bakteriellem Befall, Konkrementen, Zahnstein und anderen fluoreszierenden Substanzen an Zähnen, mit
Mitteln (2) zum Erzeugen einer Anregungsstrahlung (A), welche auf einen zu untersuchenden Zahngewebebereich zu lenken ist, und
Erfassungsmitteln (7) und Auswertemitteln zum Erfassen und Bewerten einer von dem bestrahlten Zahngewebebereich als Antwort auf die Bestrahlung erzeugten Fluoreszenzstrahlung (F),
wobei in dem optischen Weg zwischen den Mittel (2) zum Erzeugen der Anregungsstrahlung (A) und dem zu untersuchenden Zahngewebebereich ein Strahlteiler (4) vorgesehen ist, der die Anregungsstrahlung (A) in Richtung des Zahngewebebereichs reflektiert und die Fluoreszenzstrahlung (F) im wesentlichen durchläßt,
**dadurch gekennzeichnet,**
**daß** der Strahlteiler durch die plane Rückseite (4) einer im wesentlichen hemisphärischen Linse (3) gebildet wird.

2. Vorrichtung zum Erkennen von Karies, Plaque, bakteriellem Befall, Konkrementen, Zahnstein und anderen fluoreszierenden Substanzen an Zähnen, mit
Mitteln (2) zum Erzeugen einer Anregungsstrahlung (A), welche auf einen zu untersuchenden Zahngewebebereich zu lenken ist, und
Erfassungsmitteln (7) und Auswertemitteln zum Erfassen und Bewerten einer von dem bestrahlten Zahngewebebereich als Antwort auf die Bestrahlung erzeugten Fluoreszenzstrahlung (F), wobei in dem optischen Weg zwischen den Mittel zum Erzeugen (2) der Anregungsstrahlung (A) und dem zu untersuchenden Zahngewebebereich ein Strahlteiler (4) vorgesehen ist, der die Anregungsstrahlung (A) in Richtung des Zahngewebebereichs reflektiert und die Fluoreszenzstrahlung (F) im wesentlichen durchläßt,
**dadurch gekennzeichnet,**
**daß** die Mittel (2) zum Erzeugen der Anregungsstrahlung (A) zusammen mit dem Strahlteiler (4) in ein zahnärztliches Handstück (1, 10, 30) integriert sind.

3. Vorrichtung nach Anspruch 2,
**dadurch gekennzeichnet,**
**daß** der Strahlteiler durch die plane Rückseite (4) einer im wesentlichen hemisphärischen Linse (3) gebildet wird.

4. Vorrichtung nach einem der Ansprüche 1 bis 3,
**dadurch gekennzeichnet,**
**daß** diese eine Sonde (11) zur Parodontaldiagnostik aufweist, welche in eine Zahntasche eines zu untersuchenden Zahnes einzuführen ist und an deren Sondenspitze (17) die Anregungsstrahlung (A) ausgekoppelt und auf den zu untersuchenden Zahngewebebereich gelenkt wird.

5. Vorrichtung nach Anspruch 4,
**dadurch gekennzeichnet,**
**daß** die Sonde (11) aus einem lichtleitenden Material besteht und die Form einer Parodontalsonde, eines Kegels oder eines Lichtkeils hat.

6. Vorrichtung nach Anspruch 4 oder 5,
**dadurch gekennzeichnet,**
**daß** die Sonde (11) an ihrer Außenseite Markierungen (21) oder eine Skala aufweist.

7. Vorrichtung nach einem der Ansprüche 4 bis 6,
**dadurch gekennzeichnet,**
**daß** die Sonde (11) in Axialrichtung federnd gelagert ist.

8. Vorrichtung nach einem der Ansprüche 4 bis 7,
**dadurch gekennzeichnet,**
**daß** diese einen flexiblen Lichtleiter (19) aufweist, um die Anregungsstrahlung (A) in das der Sondenspitze (17) gegenüberliegende Ende der Sonde (11) einzukoppeln.

9. Vorrichtung nach einem der Ansprüche 4 bis 7,
**dadurch gekennzeichnet,**
**daß** diese einen Spiegel (20) aufweist, der in dem optischen Weg zwischen den Mitteln (2) zum Erzeugen der Anregungsstrahlung (A) und der Sonde (11) angeordnet ist, um die Anregungsstrahlung (A) in das der Sondenspitze (17) gegenüberliegende Ende der Sonde (11) einzukoppeln.

10. Zahnmedizinische Vorrichtung mit einem Handstück mit einer Instrumentenspitze (32, 40) zur mechanischen Bearbeitung eines Zahngewebebereichs,
wobei die Vorrichtung ferner aufweist:
Mittel (2) zum Erzeugen einer Anregungsstrahlung (A), welche auf den durch die Instrumentenspitze (32, 40) zu bearbeitenden Zahngewebebereich zu lenken ist,
Erfassungsmittel (7) zum Erfassen einer an dem bestrahlten und zu bearbeitenden Zahngewebebereich als Antwort auf die Bestrahlung erzeugten Fluoreszenzstrahlung (F) sowie
Auswertemittel zur Diagnose des Zustands des bestrahlten und zu bearbeitenden Zahngewebebereichs anhand der Intensität der Fluoreszenzstrahlung (F).

11. Zahnmedizinische Vorrichtung nach Anspruch 10,
**dadurch gekennzeichnet,**
**daß** diese eine optische oder akustische Anzeigevorrichtung zur Anzeige der Intensität der Fluoreszenzstrahlung (F) oder des Diagnoseergebnisses aufweist.

12. Zahnmedizinische Vorrichtung nach Anspruch 10 oder 11,
**dadurch gekennzeichnet,**
**daß** diese eine Steuereinrichtung aufweist welche die mechanische Bearbeitung des Zahngewebebereichs durch die Instrumentenspitze (32, 40) in Abhängigkeit von dem Diagnoseergebnis steuert.

13. Zahnmedizinische Vorrichtung nach einem der Ansprüche 10 bis 12,
**dadurch gekennzeichnet,**
**daß** diese einen innerhalb oder an der Außenseite der Instrumentenspitze (32, 40) verlaufenden Lichtleiter (33, 42) aufweist, um die Anregungsstrahlung (A) auf den zu bearbeitenden Zahngewebebereich zu lenken.

14. Zahnmedizinische Vorrichtung nach Anspruch 13,
**dadurch gekennzeichnet,**
**daß** die Fluoreszenzstrahlung (F) über den selben Lichtleiter (33, 42) von der Instrumentenspitze (32, 40) zu den Erfassungsmitteln (7) übertragen wird.

15. Zahnmedizinische Vorrichtung nach einem der Ansprüche 10 bis 14,
**dadurch gekennzeichnet,**
daß in dem optischen Weg zwischen den Mittel (2) zum Erzeugen der Anregungsstrahlung (A) und dem zu untersuchenden Zahngewebebereich ein Strahlteiler vorgesehen ist, der die Anregungsstrahlung (A) in Richtung des Zahngewebebereichs reflektiert und die Fluoreszenzstrahlung (F) im wesentlichen durchläßt,
wobei dieser Strahlteiler durch die plane Rückseite (4) einer im wesentlichen hemisphärischen Linse (3) gebildet wird.

16. Zahnmedizinische Vorrichtung nach einem der Ansprüche 10 bis 15,
**dadurch gekennzeichnet,**
**daß** diese einen Schwingungserzeuger (31) aufweist, durch den die Instrumentenspitze (32) zur mechanischen Bearbeitung des Zahngewebebereichs in Schwingungen versetzt wird.

17. Zahnmedizinische Vorrichtung nach einem der Ansprüche 10 bis 15,
**dadurch gekennzeichnet,**
**daß** die Instrumentenspitze (40) eine Zuführungsleitung (41) aufweist, über die ein abrasives Behandlungsmittel auf den zu bearbeitenden Zahngewebebereich gelenkt wird.

18. Vorrichtung nach einem der vorherigen Ansprüche,
**dadurch gekennzeichnet,**
**daß** die plane Rückseite (4) der hemisphärischen Linse (3) eine Beschichtung aufweist, welche die Anregungsstrahlung (A) reflektiert und die Fluoreszenzstrahlung (F) im wesentlichen durchläßt.

19. Vorrichtung nach einem der vorherigen Ansprüche,
**dadurch gekennzeichnet,**
**daß** in dem optischen Weg zwischen der hemisphärischen Linse (3) und den Erfassungsmitteln (7) ein lediglich für die Fluoreszenzstrahlung (F) durchlässiges Filter (5) angeordnet ist.

20. Vorrichtung nach einem der vorherigen Ansprüche,
**dadurch gekennzeichnet,**
**daß** die Mittel zum Erzeugen der Anregungsstrahlung (A) eine Laserdiode (2) aufweisen.

21. Vorrichtung nach einem der vorherigen Ansprüche,
**dadurch gekennzeichnet,**
**daß** die Erfassungsmittel eine Photodiode (7) aufweisen.
